(19) **Europäisches Patentamt / European Patent Office / Office européen des brevets**

(11) **EP 3 100 677 A1**

(12) **EUROPEAN PATENT APPLICATION**
published in accordance with Art. 153(4) EPC

(43) Date of publication:
**07.12.2016 Bulletin 2016/49**

(21) Application number: **15743084.4**

(22) Date of filing: **27.01.2015**

(51) Int Cl.:
**A61B 5/0408** *(2006.01)*    **A41D 13/12** *(2006.01)*

(86) International application number:
**PCT/JP2015/052229**

(87) International publication number:
**WO 2015/115441 (06.08.2015 Gazette 2015/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **28.01.2014 JP 2014013788**

(71) Applicants:
- **Nippon Telegraph and Telephone Corporation**
  **Tokyo 100-8116 (JP)**
- **Toray Industries, Inc.**
  **Tokyo 103-8666 (JP)**

(72) Inventors:
- **TSUKADA, Shingo**
  **Musashino-shi**
  **Tokyo 180-8585 (JP)**
- **KASAI, Nahoko**
  **Musashino-shi**
  **Tokyo 180-8585 (JP)**
- **SUMITOMO, Koji**
  **Musashino-shi**
  **Tokyo 180-8585 (JP)**
- **TAKAGAHARA, Kazuhiko**
  **Musashino-shi**
  **Tokyo 180-8585 (JP)**
- **ONO, Kazuyoshi**
  **Musashino-shi**
  **Tokyo 180-8585 (JP)**
- **KAWANO, Ryusuke**
  **Musashino-shi**
  **Tokyo 180-8585 (JP)**
- **ISHIHARA, Takako**
  **Musashino-shi**
  **Tokyo 180-8585 (JP)**
- **KOIZUMI, Hiroshi**
  **Musashino-shi**
  **Tokyo 180-8585 (JP)**
- **ODA, Naoki**
  **Tokyo 103-8666 (JP)**
- **TAKEDA, Keiji**
  **Otsu-shi**
  **Shiga 520-2141 (JP)**
- **ISHIKAWA, Emiko**
  **Osaka-shi**
  **Osaka 530-8222 (JP)**
- **NAGAI, Noriko**
  **Otsu-shi**
  **Shiga 520-2141 (JP)**
- **TESHIGAWARA, Takashi**
  **Tokyo 103-8666 (JP)**

(74) Representative: **Webster, Jeremy Mark et al**
**Mewburn Ellis LLP**
**City Tower**
**40 Basinghall Street**
**London EC2V 5DE (GB)**

(54) **VITAL SIGN DETECTION GARMENT**

(57)    Provided is a biosignal detecting garment that can stably detect biosignals over a long period of time without giving wearers a feeling of discomfort. The biosignal detecting garment of the present invention includes at least two or more electrodes each including a conductive fiber structure, a measurement device configured to detect and process a bioelectric signal acquired by the electrodes that are in contact with a living body, a wiring portion conductively connecting the electrodes to the measurement device, and a garment body on which the electrodes, the measurement device, and the wiring portion are placed at predetermined positions.

EP 3 100 677 A1

# FIG.1

## Description

Field

The present invention relates to a biosignal detecting garment for measuring bioelectric signals including electrocardiograms.

Background

Bio-electrodes attached on the body surface are widely used for recording bioelectric signals such as brain waves, event-related potential evoked potentials, electromyograms, and electrocardiograms and for electric stimulation of living bodies. It has been known in recent years that signs of autonomic imbalance and heart disease can be detected early by recording cardiographic waveforms over a long period of time and analyzing changes in the waveforms, which is effective in preventive medicine, as an individual health management method. Clothing on which bio-electrodes are attached (wearable electrodes) are attracting attention to acquire cardiographic waveforms over a long period of time (see Non Patent Literature 1).

Citation List

Non Patent Literature

Non Patent Literature 1: David M. D. Ribeiro, et al., "A Real time, Wearable ECG and Continuos Blood Pressure Monitoring System for First Responders", 33rd Annual International Conference of the IEEE EMBS, pp. 6894-6898, 2011

Summary

Technical Problem

However, conventional wearable electrodes have had problems in that it is difficult to perform long-term measurement because of electrodes placed at positions difficult to have contact with living bodies, that the wires extending from the electrodes to biosignal measurement devices are not integrated with the clothing and give wearers a feeling of discomfort, and that signals are degraded due to movements of the wires. In addition, conventional wearable electrodes have had problems in that the electrodes are not moisture-permeable and are likely to cause a stuffy feeling on the skin due to sweating, resulting in giving wearers a feeling of discomfort. In addition, there has been a problem in that it is difficult to obtain stable conductivity in a condition with no sweating because the skin and the electrodes are dry.

In view of the above problems, the present invention has an object to provide a biosignal detecting garment that can stably detect biosignals over a long period of time without giving wearers a feeling of discomfort.

Solution to Problem

To solve the above-described problem and achieve the above-described object, a biosignal detecting garment according to the present invention includes: at least two or more electrodes each including a conductive fiber structure; a measurement device configured to detect and process a bioelectric signal acquired by the electrodes in contact with a living body; a wiring portion conductively connecting the electrodes to the measurement device; and a garment body on which the electrodes, the measurement device, and the wiring portion are placed at predetermined positions.

In the biosignal detecting garment according to the present invention, the measurement device is an electrocardiogram measurement device. Any one of the electrodes is used as a different electrode, at least one electrodes other than the different electrode is used as at least one indifferent electrode (reference biopotential electrode), and a potential difference between the different electrode and the indifferent electrode is detected as an electrocardiographic waveform.

In the biosignal detecting garment according to the present invention, the measurement device is an electrocardiogram measurement device. The number of the electrodes each including the conductive fiber structure is at least three or more, any two of the electrodes are used as different electrodes, at least one electrode other than the different electrodes is used as at least one indifferent electrode (reference biopotential electrode), and a potential difference between the two different electrodes is detected as an electrocardiographic waveform.

In the biosignal detecting garment according to the present invention, the measurement device is an electrocardiogram measurement device. Two of the electrodes are respectively placed at about right and left sides of a chest or a flank of the garment body, and when three or more of the electrodes are included, rest of the electrodes is placed at a position separated from the electrodes placed at about the right and left sides of the chest or the flank of the garment body.

In the biosignal detecting garment according to the present invention, the electrodes respectively placed at about the right and left sides of the chest or the flank of the garment body are used as two different electrodes, the rest of the electrode is used as at least one indifferent electrode (reference biopotential electrode), and a potential difference between the two different electrodes is detected as an electrocardiographic waveform.

In the biosignal detecting garment according to the present invention, the conductive fiber structure is a fiber structure impregnated with a conductive polymer.

In the biosignal detecting garment according to the present invention, a dispersion in which the conductive polymer and a binder are dispersed in a solvent is applied to the conductive fiber structure to impregnate the fiber structure with the conductive polymer.

In the biosignal detecting garment according to the present invention, the conductive polymer is a mixture of a poly(3,4-ethylenedioxythiophene) and a polystyrenesulfonic acid.

In the biosignal detecting garment according to the present invention, a fiber structure used for the electrodes includes a woven or knitted fabric having an areal weight of equal to or more than 50 $g/m^2$ and equal to or less than 300 $g/m^2$.

In the biosignal detecting garment according to the present invention, a woven or knitted fabric used for the electrodes includes a synthetic fiber multifilament at least part of which has a fineness of equal to or more than 30 dtex and equal to or less than 400 dtex and a single-yarn fineness of equal to or less than 0.2 dtex.

In the biosignal detecting garment according to the present invention, a woven or knitted fabric used for the electrodes includes a synthetic fiber multifilament at least part of which has a single-yarn diameter of equal to or more than 10 nm and equal to or less than 5,000 nm.

In the biosignal detecting garment according to the present invention, a woven or knitted fabric used for the electrodes includes a synthetic fiber multifilament at least part of which has a single-yarn diameter of equal to or more than 10 nm and equal to or less than 1,000 nm.

The biosignal detecting garment according to the present invention further includes a resin layer that is layered on a face of the conductive fiber structure used for the electrodes, the face being opposite to another face configured to have contact with skin.

In the biosignal detecting garment according to the present invention, the resin layer includes a polyurethane-based moisture-permeable layer.

In the biosignal detecting garment according to the present invention, the wiring portion is formed of a printed conductive resin, a laminated conductive resin film, a conductive fiber, or a metal wire.

In the biosignal detecting garment according to the present invention, the wiring portion is formed by sewing-in of a conductive fiber, the conductive fiber comprising a fiber coated with a metal.

In the biosignal detecting garment according to the present invention, the metal with which the conductive fiber is coated includes silver, aluminum, or stainless steel.

In the biosignal detecting garment according to the present invention, the wiring portion is disposed on an outer side of the garment body.

In the biosignal detecting garment according to the present invention, the wiring portion is formed by sewing-in of a conductive fiber, the conductive fiber being sewn in as one thread of a sewing machine by sewing so as to be exposed mainly on an outer side of the garment body.

In the biosignal detecting garment according to the present invention, the wiring portion is disposed on an outer side of the garment body, and part of the wiring portion exposed on the outer side of the garment body is covered with a waterproof electric insulating member.

In the biosignal detecting garment according to the present invention, the electric insulating member includes a polyurethane-based film.

In the biosignal detecting garment according to the present invention, the wiring portion is formed of a conductive resin. The wiring portion is formed with the conductive resin being continuously layered on part of one face of a sheet including a waterproof electric insulating member, and with the face of the waterproof electric insulating member on which the conductive resin is layered being bonded to the garment body.

The biosignal detecting garment according to the present invention includes at least two conductive connection systems. Each of the conductive connection systems includes: one of the electrodes; the measurement device; and the wiring portion conductively connecting the electrode to the measurement device. At least parts of the conductive connection systems formed on the garment body are separated from each other by a water-repellent and insulating structure.

In the biosignal detecting garment according to the present invention, the garment body includes a woven or knitted fabric having a stress of equal to or more than 0.5 N and equal to or less than 15 N at an elongation of 60% in a length or breadth direction. The electrodes are closely attached to skin at a pressure of equal to or more than 0.1 kPa and equal to or less than 2.0 kPa when being worn.

In the biosignal detecting garment according to the present invention, the garment body includes a woven or knitted fabric including elastic yarn and inelastic yarn.

In the biosignal detecting garment according to the present invention, the elastic yarn includes a polyurethane-based elastic fiber.

In the biosignal detecting garment according to the present invention, the garment body includes a knitted fabric.

In the biosignal detecting garment according to the present invention, the measurement device is configured to be attached and connected to and detached from the garment body via a connector.

In the biosignal detecting garment according to the present invention, the measurement device has a function of transferring data through communication with at least one of a mobile terminal and a personal computer.

In the biosignal detecting garment according to the present invention, the measurement device has a function of transferring data through wireless communication with at least one of a mobile terminal and a personal computer.

Moreover, a biosignal detecting garment according to the present invention includes: at least two or more electrodes each including a conductive fiber structure; a connector through which a measurement device configured to detect and process a bioelectric signal acquired by the electrodes that are in contact with a living body is configured to be attached, connected, and detached; a wiring portion conductively connecting the electrodes to the connector; and a garment body on which the electrodes, the connector, and the wiring portion are placed at predetermined positions.

Advantageous Effects of Invention

The biosignal detecting garment according to the present invention can continuously and stably detect biosignals over a long period of time without giving wearers a feeling of discomfort while being worn by placing electrodes, wiring portions, and a measurement device at predetermined positions on a garment body.

Brief Description of Drawings

FIG. 1 is a schematic diagram of a biosignal detecting garment according to an embodiment of the present invention.
FIG. 2 is a cross-sectional view of the biosignal detecting garment illustrated in FIG. 1 along the line A-A'.
FIG. 3 is a schematic diagram of a biosignal detecting garment according to a modification of the embodiment of the present invention.
FIG. 4 is a schematic block diagram of a measurement device according to the embodiment of the present invention.
Description of Embodiments

A biosignal detecting garment according to the present invention will be described below in detail on the basis of the drawings. The embodiments do not limit the present invention.

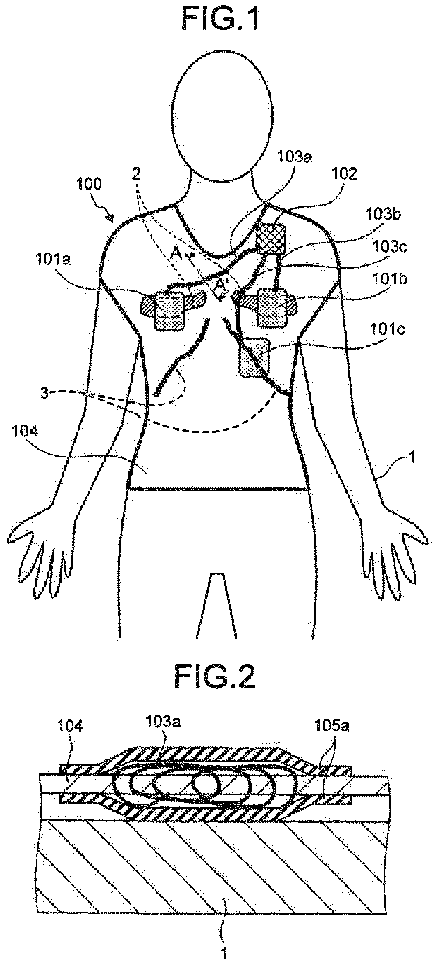

FIG. 1 is a schematic diagram of a biosignal detecting garment according to an embodiment of the present invention. As illustrated in FIG. 1, a biosignal detecting garment 100 of the present invention includes three electrodes 101a, 101b, and 101c each including a conductive fiber structure, a measurement device 102 configured to detect and process bioelectric signals acquired by the electrodes 101a, 101b, and 101c, wiring portions 103a, 103b, and 103c conductively connecting the respective electrodes 101a, 101b, and 101c to the measurement device 102, and a garment body 104 on which the electrodes 101a, 101b, and 101c, the measurement device 102, and the wiring portions 103a, 103b, and 103c are placed at predetermined positions. An example in which the measurement device 102 is an electrocardiogram measurement device will be described below.

In the biosignal detecting garment 100, the electrodes 101a and 101b are respectively placed on portions configured to have contact with about right and left sides of the chest or the flank when the garment is worn on the inner face (the face configured to have contact with a body 1) of the garment body 104, and the electrode 101c is placed at a position below the electrode 101b, the position being separated from the electrodes 101a and 101b placed at about the right and left sides of the chest or the flank of the garment body 104.

Since the three electrodes 101a, 101b, and 101c are placed at about the right and left sides of the chest or the flank of the garment body 104 and a site separated from about the right and left sides of the chest or the flank, the electrodes 101a, 101b, and 101c can have a stable contact with the body 1 and enables long-term continuous measurement of biosignals. The measurement device 102 is conductively connected with the electrodes 101a, 101b, and 101c by the wiring portions 103a, 103b, and 103c directly placed on the garment body 104. The wiring portions 103a, 103b, and 103c are integrated with the garment body 104, and thus signal degradation due to movements of the wires 103a, 103b, and 103c can be prevented without giving wearers a feeling of discomfort.

In the biosignal detecting garment 100, the electrodes 101a and 101b are used as two different electrodes, and 101c is used as a indifferent electrode (a reference biopotential electrode), so that the potential difference between the different electrodes 101a and 101b is detected as an electrocardiographic waveform. Biosignals can be detected using the electrode 101a placed at about the right chest or the left flank as a positive different electrode and the electrode 101b placed at about the left chest or the left flank as a negative different electrode. Using the electrode 101b as the positive different electrode and the electrode 101a as the negative different electrode is advantageous to automatic analysis of pulse intervals (R-R intervals) and the like because detection can be performed so that the amplitudes of QRS signals will be large.

FIG. 1 illustrates a case in which three electrodes are used, but the number is not limited to three as long as the number is equal to or more than two. When two electrodes are used, the electrodes may be respectively placed at about the right and left sides of the chest or the flank of the garment body 104, any one of the electrodes may be used as the different electrode, and the electrode other than the different electrode may be used as the indifferent electrode (the reference biopotential electrode) to detect the potential difference between the different electrode and the indifferent electrode as an electrocardiographic waveform. When equal to or more than four electrodes are used, any two of the electrodes may be used as the different electrodes and the electrodes other than the different electrodes may be used as the indifferent electrodes (the reference biopotential electrodes) to detect the potential difference between the different electrodes as an electrocardiographic waveform. When equal to or more than four electrodes are used, two of the electrodes are preferably placed at about the right and left sides of the chest or the flank of the garment body 104, and the other electrodes are preferably placed at positions separated from the two electrodes placed at about the right and left sides of the chest or the flank of the garment body 104.

In the biosignal detecting garment 100 of the present invention, each of the electrodes 101 (101a, 101b, and 101c) configured to detect biosignal from the body 1 is preferably a fiber structure impregnated with a conductive polymer. More preferably, a conductive resin is supported on the surfaces of filaments constituting the fiber structure and/or in the gaps between the filaments. Each of the electrodes 101 is preferably a fiber structure because the conductive resin can be supported between the filaments constituting the fiber structure. It has been conventionally inevitable to apply an acrylic gel to the surfaces of electrodes to enhance adhesion to the body 1 and obtain electric signals when using film electrodes commonly used as electrodes for electrocardiograms, which has the problem that skin problems are likely to happen.

On the other hand, the electrodes 101 according to the present invention each including the fiber structure are less irritating when being in contact with the skin and are safe. When signals cannot be obtained well due to dry skin, good electrocardiograms can be obtained by applying a small quantity of a physiological saline solution or a moisturizer to the fiber structure. Examples of the moisturizer used include glycerol, sorbitol, polyethylene glycols, polyethylene glycol-polypropylene glycol copolymers, ethylene glycol, sphingosine, and phosphatidylcholines. One of these examples may be used singly, or equal to or more than two may be used in combination. These moisturizers are also used as moisturizing ingredients in cosmetics and are highly safe for the skin.

The conductive polymer used for the electrodes 101 according to the present invention is not limited as long as the polymer is a conductive resin. Conductive resin compositions in which carbon black, carbon nanotubes (CNTs), metal nanoparticles, or other substances are blended in resins with low conductivities may be used, but conductive polymers in which the resins themselves have conductivity are preferable.

The conductive polymer is not limited to particular materials as long as the polymer is conductive. Examples of the conductive polymer include acetylene-based conductive polymers; 5-membered heterocycle-based conductive polymers such as pyrrole-based polymers including polypyrroles, poly(3-alkylpyrrole)s such as a poly(3-methylpyrrole), a poly(3-ethylpyrrole), and a poly(3-dodecylpyrrole), poly(3,4-dialkylpyrrole)s such as a poly(3,4-dimethylpyrrole) and a poly(3-methyl-4-dodecylpyrrole), poly(N-alkylpyrrole)s such as a poly(N-methylpyrrole) and a poly(N-dodecylpyrrole), poly(N-alkyl-3-alkylpyrrole)s such as a poly(N-methyl-3-methylpyrrole) and a poly(N-ethyl-3-dodecylpyrrole), and poly(3-carboxypyrrole)s; thiophene-based polymers including polythiophenes, poly(3-alkylthiophene)s such as a poly(3-methylthiophene), a poly(3-ethylthiophene), and a poly(3-dodecylthiophene), poly(3,4-dialkylthiophene)s such as a poly(3,4-dimethylthiophene) and a poly(3-methyl-4-dodecylthiophene), poly(3-alkoxythiophene)s such as a poly(3-hydroxythiophene) and a poly(3-methoxythiophene), poly(3,4-dialkylthiophene)s such as a poly(3,4-dimethylthiophene) and poly(3,4-dibutylthiophene), poly(3-carboxythiophene)s, poly(3-halothiophene)s such as a poly(3-bromothiophene) and a poly(3-chlorothiophene), and poly(3,4-ethylenedioxythiophene)s; and isothianaphthene-based polymers; aniline-based conductive polymers such as a polyaniline, a poly(2-methylaniline), and a poly(3-isobutylaniline); and phenylene-based conductive polymers such as poly(p-phenylenevinylene)s (PPVs), and copolymers of these polymers. Use of a dopant in combination improves the conductivity of the conductive polymer. The dopant used in combination with the conductive polymer is at least one kind of ions including halide ions such as chloride ions and bromide ions; perchlorate ions; tetrafluoroborate ions; hexafluoroarsenate ions; sulfate ions; nitrate ions; thiocyanate ions; hexafluorosilicate ions; phosphate-based ions such as phosphate ions, phenylphosphate ions, and hexafluorophosphate ions; trifluoroacetate ions; tosylate ions; alkylbenzenesulfonate ions such as ethylbenzenesulfonate ions and dodecylbenzenesulfonate ions; alkylsulfonate ions such as methylsulfonate ions and ethylsulfonate ions; and polymer ions such as polyacrylate ions, polyvinylsulfonate ions, polystyrenesulfonate ions, and poly(2-acrylamido-2-methylpropanesulfonate) ions. The amount of the dopant to be added is not limited to particular values as long as the quantity is sufficient to affect the conductivity.

As the conductive polymer, among the above polymers, a polypyrrole, a poly(3,4-ethylenedioxythiophene) (PEDOT), a polyaniline, a poly(p-phenylenevinylene) (PPV) and the like are easy to resinify and are preferably used in the form of conductive resins. PEDOT/PSS, which is produced by doping PEDOT, a thiophene-based conductive polymer, with a poly(styrenesulfonic acid) (poly(4-styrenesulfonate): PSS), is particularly preferable in terms of safety and workability. In terms of enhancing the conductivity and stabilizing, adding glycerol, a physiological saline solution, or other substances to the fiber structure containing the conductive polymer can be preferably used.

In addition, the fiber structure is preferably impregnated with the conductive polymer such as PEDOT/PSS by applying a dispersion in which the polymer and a binder are dispersed in a solvent to the fiber structure or by dipping the fiber structure in the dispersion. Use of the binder in combination with the conductive polymer enhances scratch resistance and surface hardness of a coating containing the conductive polymer and enhances adhesion to base materials.

Using the binder can cause the conductive polymer to be easily supported on the fiber structure and can prevent the surface resistance from rising after repeated washing of electrode materials.

The binder may be a thermosetting resin or may be a thermoplastic resin. Examples include polyesters such as a polyethylene terephthalate, a polybutylene terephthalate, and a polyethylene naphthalate; polyimides; polyamideimides; polyamides such as a polyamide 6, a polyamide 6,6, a polyamide 12, and a polyamide 11; fluororesins such as a polyvinylidene fluoride, a polyvinyl fluoride, a polytetrafluoroethylene, an ethylene/tetrafluoroethylene copolymer, and a polychlorotrifluoroethylene; vinyl resins such as a polyvinyl alcohol, a polyvinyl ether, a polyvinyl butyral, a polyvinyl acetate, and a polyvinyl chloride; epoxy resins; xylene resins; aramid resins; polyimide silicones; polyurethanes; polyureas; melamine resins; phenolic resins; polyethers; acrylic resins; and copolymers of these polymers. These binders may be dissolved in an organic solvent, may be functionalized with groups such as sulfonate group or carboxy group to form an aqueous solution, or may be dispersed in water by emulsification, for example.

Among the binder resins, preferable resins are at least one of polyurethanes, polyesters, acrylic resins, polyamides, polyimides, epoxy resins, and polyimide silicones because these polymers can be easily mixed.

The solvent used is not limited as long as the conductive polymer and the binder can be stably dispersed, and water or a mixed solution of water and an alcohol can be preferably used. When a polythiophene-based conductive polymer such as PEDOT/PSS is used, a mixed solvent of water and ethanol is preferable.

Examples of the form of the fiber structure used for each of the electrodes 101 include woven fabric, knitted fabric, and nonwoven fabric. The areal weight of the fiber structure is preferably equal to or more than 50 g/m$^2$ and equal to or less than 300 g/m$^2$ because a lack in the quantity of the conductive resin with which the fiber structure is impregnated results in poor washing durability in repeated use. Less than 50 g/m$^2$ causes the impregnation quantity of the conductive resin to be small and results in poor washing durability. More than 300 g/m$^2$ causes the substantial areal weight to be large and may cause uncomfortable wearing. Equal to or more than 60 g/m$^2$ and equal to or less than 250 g/m$^2$ is more preferable. The thickness of the fiber structure is preferably equal to or more than 0.2 mm and equal to or less than 2.0 mm. When the thickness is less than 0.2 mm, the substantial areal weight is small due to the too small thickness of the cloth, and the impregnation quantity of the conductive resin is small. A thickness of larger than 2.0 mm may cause uncomfortable wearing due to the too large thickness.

Equal to or more than 0.3 mm and equal to or less than 1.5 mm is more preferable.

The sizes and the shapes of the electrodes 101 are not particularly specified as long as biosignals can be detected, and each of the length and the breadth is preferably equal to or more than 2 cm and equal to or less than 20 cm. When the length or the breadth of each of the electrodes 101 are equal to or less than 2 cm, the area of the electrode is too small, which results in a higher possibility of sliding of the electrode along with movements of the cloth during exercise or the like and a resulting higher possibility of picking up noise. Equal to or more than 20 cm is larger than the size substantially required for detecting signals and results in a too large area of the electrode, which is likely to cause troubles such as a short circuit due to a short distance between adjacent electrodes. Each of the length and the breadth is more preferably equal to or more than 2.5 cm and equal to or less than 18 cm.

To continuously obtain good cardiographic waveforms, the electrodes 101 are required to be kept in contact with and attached to the skin. Flexibility of the cloth constituting the fiber structure is required for keeping the electrodes 101 continuously attached to the skin, and thus the fiber structure is preferably woven fabric, knitted fabric, or nonwoven fabric and more preferably knitted fabric, which has higher flexibility.

In addition, the structure and a producing method of the fiber structure typified by knitted fabric are not limited to particular structures or methods. Shapes that can retain moisture such as sweat as electrodes are preferable, and multilayer knitted fabric can be preferably used among knitted fabrics. Examples of the structure include, but are not limited to, a double raschel structure, a three-layer corrugated structure, a reversible structure, an interlock structure, a circular rib structure, and a fleecy structure.

In terms of supporting of the conductive resin on the fiber structure and high conductivity, the woven or knitted fabric used for the electrodes 101 of the present invention preferably contains multifilament yarn constituted of a plurality of filaments. The fineness of the multifilament yarn is not limited to particular values and is preferably 30 dtex to 400 dtex in terms of utilizing properties as a fiber structure. The mixing ratio of the multifilament yarn in the woven or knitted fabric is not limited to particular values to the extent that the performances are not affected. The mixing ratio is preferably high in terms of conductivity and durability and is more preferably equal to or more than 50% and equal to or less than 100%.

Examples of the material of the multifilament yarn used for the woven or knitted fabric include polyester-based synthetic fibers such as polyethylene terephthalate fibers, polytrimethylene terephthalate fibers, and polybutylene terephthalate fibers, and polyamide-based synthetic fibers such as nylon fibers. Fibers containing additives such as titanium oxides may be used, and fibers polymer-modified to impart functionalities such as enhanced hygroscopic properties may be used. The cross-sectional shape of a filament unit constituting the multifilament is also not specified, and various modified cross-section yarns typified by round shapes, triangular shapes, octalobal shapes, flat shapes, and Y shapes can be used.

As inelastic yarn, sheath-core or side-by-side conjugated yarn containing polymers having different viscosities can also be used. In addition, false-twisted yarn obtained by false-twisting these original yarns may be used. Furthermore, synthetic fibers such as polyacrylonitrile and polypropylene, regenerated fibers such as rayon, polynosic, and cuprammonium rayon, and semisynthetic fibers such as acetate fibers and triacetate fibers may be used.

The fiber structure according to the present invention preferably contains a multifilament having a fiber diameter of the filament of equal to or less than 0.2 dtex in terms of supporting of the conductive resin on the surface of the filament and in the gaps between the filaments. The mixing ratio of the filament multifilament of equal to or less than 0.2 dtex in the fiber structure is not limited to particular values to the extent that the performances are not affected. The mixing ratio is preferably high in terms of conductivity and durability and is more preferably equal to or more than 50% and equal to or less than 100%. In addition, as the number of the filaments increases, the gaps formed by the filaments, in other words, sites on which the conductive resin is supported, are redifferentiated, and the conductive resin is well supported on the fiber structure. At the same time, a small fiber diameter enables continuity of the conductive resin to be retained even when redifferentiated. Thus, superior high conductivity and washing durability can be obtained. It is preferable to use microfibers having fiber diameters of equal to or less than 5 $\mu$m used for artificial leather, materials for outerwear, and other materials, and it is more preferable to use nanofibers having fiber diameters of equal to or more than 10 nm and equal to or less than 5,000 nm, particularly nanofibers having fiber diameters of equal to or more than 10 nm and equal to or less than 1,000 nm, used in recent years for liners for sports clothing, brassieres, golf gloves, and other clothing in order to prevent slipping. Fiber structures containing nanofibers produced by known methods such as aggregates of nanofiber staple yarn made of "nanoalloy (registered trademark)" fibers and aggregates of monofilament yarn made by an electrospinning method or other methods can be preferably used as the nanofibers, and fiber structures containing multifilament yarn of nanofibers are more preferable. The multifilament yarn of nanofibers can be produced by a known conjugate-spinning method, for example. An example that can be effectively used is nanofiber multifilament yarn having small fiber diameter variations obtained by removing the sea components from composite fibers obtained using composite spinnerets exemplified in Japanese Patent Application Laid-open No. 2013-185283, but this example is not limiting.

In each of the electrodes 101 used in the present invention, a resin layer is preferably layered on one face of the fiber structure containing the conductive substance. In consideration of application to bio-electrodes, the resin layer is preferably layered on the face of the fiber structure used for the electrodes 101 opposite to the face configured to have contact with the skin. If the electrodes 101 are dry when detecting biosignals, stable detection of the biosignals is difficult. Thus, keeping the electrodes 101 wet to some extent is required. Covering one face of each of the electrodes 101 with the resin layer can prevent drying and enables conductivity to be stably obtained. In addition, covering one face of each of the electrodes 101 with the resin layer can reduce the conductive resin falling off during washing and can considerably suppress washing durability.

The kind and the shape of a polymer constituting the resin layer are not limited to particular kinds and shapes as long as humidity control is enabled, and a moisture-permeable layer is preferable. Complete blocking of moisture transfer causes a stuffy feeling to be strong, which leads to a feeling of discomfort while wearing and to a cause of skin rashes and the like. Examples of the moisture-permeable layer include, but are not limited to, forms obtained by layering known membranes, films, laminates, resins, and the like such as polytetrafluoroethylene (PTFE) porous membranes, non-porous membranes of hydrophilic elastomers such as hydrophilic polyester resins and polyurethane resins, and polyurethane-resin microporous membranes by a coating or lamination method. The moisture-permeable layer is preferably obtained by laminate-bonding an elastic polyurethane-resin microporous membrane by laminating in terms of followability to the fiber structure, which is the base material. In addition, to improve moisture-permeability, micropores may be formed using a punching machine or a sewing machine on the fiber structure on one face of which the resin layer has been layered.

The electrodes 101 used in the present invention preferably have surface electric resistance values of equal to or less than $1 \times 10^6\ \Omega$ after 20 repeated washing cycles by a washing method in accordance with JIS L0217 (2012) 103 method. A value exceeding $1 \times 10^6\ \Omega$ results in larger noise in electric signals and difficulty in precise measurement.

Even with electrodes 101 having surface electric resistance values exceeding $1 \times 10^6$ Ω in a dry state, measurement can be performed if substantial surface electric resistance values are reduced to equal to or less than $1 \times 10^6$ Ω when the electrodes 101 are impregnated with liquid containing electrolytes such as tap water and sweat while being worn as the bio-detecting garment 100. The biosignal detecting garment 100 of the present invention is expected to be widely used also by ordinary families and can preferably detect biosignals until after 20 washing cycles in consideration of actual wearing.

The biosignal detecting garment 100 of the present invention requires the wiring portions 103 (103a, 103b, and 103c) for transmitting biosignals obtained by the electrodes 101 to the measurement device 102. The wiring portions 103 are preferably formed by a process of printing a conductive resin on the garment body 104 or a process of laminating a film of a conductive resin and are further preferably formed of a fiber having electrical conductivity or metal wires.

When the wiring portions 103 are formed by printing a conductive resin at predetermined positions on the garment body 104, the conductive resin used is not limited to particular resins as long as the resin has conductivity. The conductive resins used for the electrodes 101 can be mentioned as examples, and products obtained by blending carbon black, carbon nanotubes, metal nanoparticles, or a mixture of these substances into an adhesive resin such as acrylic and epoxy resins can be used. The conductive resin may be printed in a form of wiring at predetermined positions on which wires are to be formed on the garment body 104 by screen printing or rotary printing, for example.

When the wiring portions 103 are formed of a fiber with electrical conductivity, a conductive fiber in which carbon black is combined and arranged in part of the core or the sheath of a polyester or a nylon in the length direction of the fiber, or metal-coated yarn in which a polyester or nylon fiber is coated with metals including silver, aluminum, or stainless steel may be used as the conductive fiber. Methods for coating a resin fiber with metals include a method in which a polyester or nylon fiber travels within a solution of dispersed fine powder of silver, aluminum, stainless steel, or other metal to coat the fiber with metal powder and then is thermally set with a heater, and a knit de knit method in which a tubular knitted fabric is produced from a nylon or polyester fiber and put in a solution of dispersed silver, aluminum, or stainless steel, the metal is fixed on the fiber by heating, and the fiber is unraveled from the knitted fabric. The stainless steel used is particularly preferably surgical stainless steel (SUS316L), which is less irritating to human bodies. In addition, when the wiring portions 103 are formed of a metal wire, a metal wire in which a stainless wire or a copper wire is covered with insulating vinyl can be used.

When the wiring portions 103 are formed by printing the conductive resin, there is a problem in durability because the conductive resin printed on the garment body 104 may break because of cracks caused by stretching of the cloth during repeated putting on and removing or exercise. When the wiring portions 103 are formed of a metal wire, there remains the problem in safety that the metal wire may stick in the body if the metal wire is broken or the treatment of the end of the metal wire is inadequate. The wiring portions 103 are preferably formed of a conductive fiber. A conductive fiber in which a fiber is coated with silver, aluminum, or stainless steel, which has a high conductivity, is more preferably used for the wiring portions 103.

A method for attaching the conductive fiber to the garment body 104 is not limited to particular methods. The conductive fiber may be sewn in the cloth of the garment body 104 with a sewing machine or may be attached to the cloth with an adhesive resin, or a film of the conductive fiber on one face of which a hot-melt adhesive is added may be used and attached by heat bonding.

The wiring portions 103 formed by printing or the like of the conductive fiber or the conductive resin are preferably placed on the outer face (the face not configured to be closely attached to the skin) of the garment body 104. Wiring on the outer face prevents the wiring portions 103 from having direct contact with the skin and prevents noise acquired by the wiring portions 103 from being mixed in biosignals detected by the electrodes 101. Thus, biosignals can be precisely measured.

As a method for attaching the conductive fiber to the garment body 104, it is more preferable in sewing with a sewing machine to use the conductive fiber as the bobbin thread and ordinary machine sewing thread as the needle thread to perform sewing with the inner face of the cloth facing upward. This method exposes the conductive fiber mainly on the outer side of the garment body 104 not configured to be closely attached to the skin. In addition, a preferable method for sewing the conductive fiber is to sew the conductive fiber in the garment body 104 by catch-stitching. Sewing the conductive fiber by catch-stitching enables the sewing-thread portions to move when the cloth stretches and to follow the cloth, and the stretching properties are not impaired.

In the biosignal detecting garment 100 of the present invention, connecting these electrodes 101 made of the conductive fiber structures with wiring portions 103 can prevent polarization. This constitution is not only preferable for detecting weak biosignals but can prevent corrosion (electrolytic corrosion), and enables continuous use over a long period of time. A method for connecting the electrodes 101 with the wiring portions 103 is not limited to particular methods, and examples of the method includes a process of sewing the electrodes 101 placed on the garment body 104 with the conductive fiber by sewing to form the wiring portions 103, a process of printing the conductive resin to form the wiring portions 103 so as to overlap the electrodes 101, and a process of adding a hot-melt adhesive on one face of each conductive resin film and bonding the films to the electrodes 101 by thermocompression bonding to form the wiring portions 103.

The wiring portions 103 of the present invention are required to transmit biosignals obtained from the electrodes 101 to the measurement device 102 with a high sensitivity, and, regarding the wiring portions 103, the wiring portions 103 exposed on the garment body 104 are preferably covered with waterproof electric insulating members and insulated from the body 1 and the outside air. This insulation enables biosignals to be measured well even with profuse sweating or a rainfall. As the waterproof electric insulating member, a waterproof film on one face of which a hot-melt adhesive is added is preferably used, and a polyurethane-based film, which is superior in elasticity, is particularly preferable in that the film does not impair followability of the cloth during putting on and removing or exercise. As a method for covering the wiring portions 103, a process of attaching the wiring portions 103 to the garment body 104 and then bonding the waterproof electric insulating members on which a hot-melt adhesive has been added by thermocompression bonding with an iron or a press from both sides of the cloth of the garment body 104 so that the wiring portions 103 will be completely covered is preferably used.

FIG. 2 is a cross-sectional view of the biosignal detecting garment 100 illustrated in FIG. 1 along the line A-A'. The wire 103a illustrated in FIG. 2 is formed by sewing a conductive fiber or a metal wire in the garment body 104. As illustrated in FIG. 2, the conductive fiber or the metal wire forming the wiring portion 103a is exposed on the outer side and the inner side of the garment body 104, and covering both faces with waterproof electric insulating members 105a is preferable. However, if at least the wiring portion 103a exposed on the side configured to have contact with the skin is covered with the electric insulating member 105a, noise from the wiring portion 103a can be removed, and biosignals can be stably detected. Exposed portions of the wiring portions 103b and 103c are also covered with electric insulating members 105b and 105c in the same manner as for the wiring portion 103a. This covering enables biosignals to be measured well even with profuse sweating or a rainfall. When the wiring portions 103 are formed by printing the conductive resin on the outer or inner face of the garment body 104, only the face on which the conductive resin has been printed may be covered with the electric insulating members 105.

It is preferable that the outer face (the face not configured to adhere to the skin) of the garment body 104 on which the electrodes 101 are attached is also covered with the waterproof electric insulating members 105 used for covering the wiring portions 103. Covering the outer face of the garment body 104 on which the electrodes 101 are placed can prevent water from permeating through the surface of the cloth when it is raining.

Each of the wiring portions 103 may be formed by continuously layering the conductive resin on part of one face of a sheet of one of the waterproof electric insulating members 105 and bonding the face of each of the waterproof electric insulating members 105 on which the conductive resin has been layered to the garment body 104. As described above, when the conductive resin is printed on the cloth, the conductive resin may break because of cracks caused by stretching of the cloth during repeated putting on and removing of the biosignal detecting garment 100 or exercise. However, when the wiring portions 103 are made by layering the conductive resin and the waterproof electric insulating members 105, the wiring portions 103 can be stably used because the waterproof electric insulating members 105 lack voids and elasticity unlike the cloth and are less likely to generate cracks in the conductive resin layer even when stretching.

In the biosignal detecting garment 100 of the present invention, each conductive connection system is constituted of one of the electrodes 101, the measurement device 102, and one of the wiring portions 103 conductively connecting the electrodes 101 to the measurement device 102. The conductive connection systems are preferably separated from each other by water-repellent and insulating structures. Separating the conductive connection systems from each other prevents a short circuit due to permeation of an electrolyte solution such as sweat into the garment body 104 and enables biosignals to be stably measured even with profuse sweating during exercise or a rainfall.

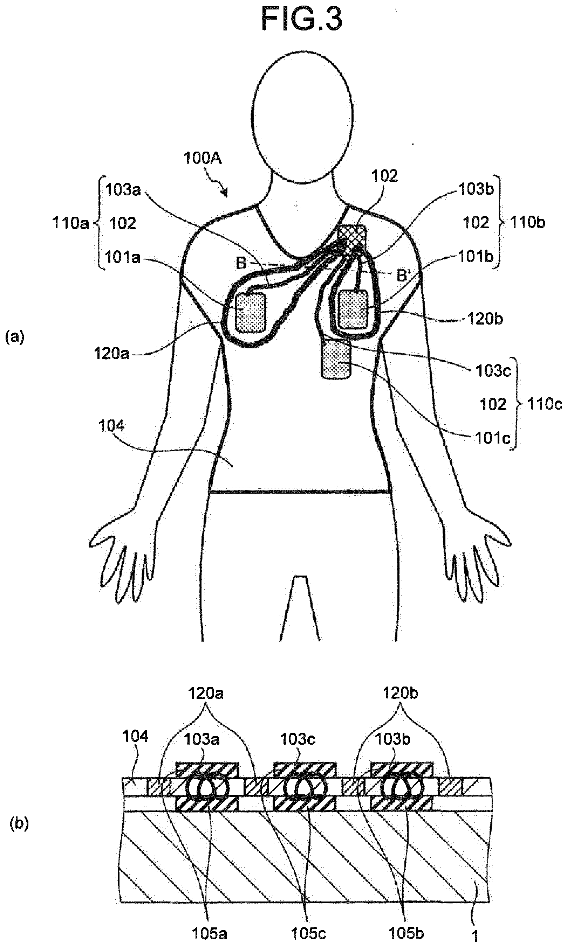

FIG. 3 is a schematic diagram of a biosignal detecting garment 100A according a modification of the embodiment of the present invention. FIG. 3(a) is a schematic front view of the biosignal detecting garment 100A when being worn, and FIG. 3(b) is a cross-sectional view of the biosignal detecting garment 100A in FIG. 3(a) along the line B-B'. As illustrated in FIG. 3, the biosignal detecting garment 100A according to the modification includes three conductive connection systems 110 constituted of the electrodes 101, the measurement device 102, and the wiring portions 103. In other words, there are a conductive connection system 110a constituted of the electrode 101a, the measurement device 102, and the wiring portion 103a, a conductive connection system 110b constituted of the electrode 101b, the measurement device 102, and the wiring portion 103b, and a conductive connection system 110c constituted of the electrode 101c, the measurement device 102, and the wiring portion 103c. The three conductive connection systems 110 are separated from each other by water-repellent and insulating structures 120. The water-repellent and insulating structures 120 include a structure 120a separating the electrode 101a and the wiring portion 103a from the other portions of the biosignal detecting garment 100A and a structure 120b separating the electrode 101b and the wiring portion 103b from the other portion. The water-repellent and insulating structures 120a and 120b separate the three conductive connection systems 110a, 110b, and 110c from each other. As illustrated in FIG. 3(b), the water-repellent and insulating structures 120 are provided so as to divide the cloth of the garment body 104 in the thickness direction and thus can prevent a short circuit to enable biosignals to be stably measured. When the water-repellent and insulating structures 120 are formed inside the cloth of the garment body 104, a fiber made of the above waterproof electric insulating members 105 may be sewn by sewing.

The water-repellent and insulating structures 120 may be formed on the outer face of the cloth of the garment body 104. In this case, sheets of the above waterproof electric insulating members 105 may be attached to both faces of the garment body 104.

When the number of the conductive connection systems 110 is two, or even when the number is equal to or more than three, the water-repellent and insulating structures 120 may separate the conductive connection systems 110 from each other.

In the biosignal detecting garments 100 and 100A of the present invention, the electrodes 101 placed on the garment body 104 are required to be closely attached to the body 1 in order to obtain signals containing less noise. The electrodes 101 are preferably closely attached to the body 1 at least at a pressure of equal to or more than 0.1 kpa and equal to or less than 2.0 kpa in terms of compression of the body 1 by the electrodes 101. A pressure exceeding 2.0 kpa enables acquisition of good signals, but the strong compression results in a stifling feeling in wearing. A pressure of equal to or less than 0.1 kpa allows the electrodes to be separated from the skin during actions and prevents good signals from being obtained. Equal to or more than 0.5 pa and equal to or less than 1.5 pa is more preferable.

To achieve this compression, is it preferable that the garment body 104 is made of woven or knitted fabric having a stress of equal to or more than 0.5 N and equal to or less than 15 N at an elongation of 60% in either the length or breadth direction of the woven or knitted fabric. The compression can be adjusted by the stretching properties and the sewing size of the cloth, but a compression less than 0.5 N may cause breakage because of thin cloth when the cloth stretches during actions even if the above range of the compression is achieved by reducing the sewing size. A compression exceeding 15 N may cause poor movability because the cloth is less likely to stretch beyond the sewing size during actions even if the above range of the compression is achieved by increasing the sewing size. Equal to or more than 1.0 N and equal to or less than 10 N is more preferable.

To achieve this compression, the garment body 104 on which the electrodes 101 are attached is preferably a woven or knitted fabric made of elastic yarn and inelastic yarn. A woven or knitted fabric made of elastic yarn and inelastic yarn has good stretching properties of the cloth and can achieve the above compression.

The material of the elastic yarn applied to an elastic warp knitted fabric is not limited to particular materials, and examples used include polyurethane elastic fibers, polyether-ester elastic fibers, polyamide elastic fibers, polyolefin elastic fibers, what is called rubber yarn, which is yarn of a natural rubber, a synthetic rubber, or a semisynthetic rubber, and specialty fibers produced by dipping a synthetic fiber in a rubber or by coating a synthetic fiber with a rubber. Among these examples, polyurethane elastic fibers are particularly preferable because the fibers are widely used for common elastic warp knitted fabric, have good knittablity, and exhibit high degrees of elongation and good recovery properties when formed into products.

The material of the inelastic yarn applied to the elastic warp knitted fabric is also not limited to particular materials, and examples used include polyester-based synthetic fibers such as polyethylene terephthalate, polytrimethylene terephthalate, and polybutylene terephthalate, and polyamide-based synthetic fibers such as nylons. As the material of the inelastic yarn, the above fibers impregnated with additives such as titanium oxides may be used, or fibers polymer-modified to impart functionalities such as enhanced hygroscopic properties may be used. The cross-sectional shape of a filament unit of the inelastic yarn is also not specified, and various modified cross-section yarns typified by round shapes, triangular shapes, octalobal shapes, flat shapes, and Y shapes can be used. As the inelastic yarn, sheath-core or side-by-side conjugated yarn including polymers having different viscosities can also be used. In addition, false-twisted yarn obtained by false-twisting these original yarns may be used. Furthermore, synthetic fibers such as polyacrylonitrile and polypropylene, regenerated fibers such as rayon, polynosic, and cuprammonium rayon, semisynthetic fibers such as acetate fibers and triacetate fibers, and natural fibers such as cotton, hemp, wool, and silk may be used in accordance with desired properties. As described above, the most appropriate material may be selected as appropriate for the inelastic yarn depending on the intended use.

A method for weaving or knitting the woven or knitted fabric using the elastic yarn and the inelastic yarn is not limited to particular methods as long as the above compression can be achieved. For example, a process of weaving a plain-weave or twill structure using, as the warp and the weft, covered string in which elastic yarn as the core is covered with inelastic yarn as the sheath may be used for woven fabric. A process of knitting a plain structure or an interlock structure out of the covered string, or bare-yarn plain knitting or bare-yarn interlock knitting that involves knitting with inelastic yarn and elastic yarn together may be used for tubular knitting, for example. In the case of warp knitting, knitting may be performed using inelastic yarn at a front reed and elastic yarn at a back reed to produce a double Denbigh structure with a front structure of 10/01 and a back structure of 01/10 or produce a half structure with a front structure of 10/23 and a back structure of 01/10. More preferably, tubular knitting or warp knitting may be employed. This type of knitting produces cloth with high elasticity that smoothly stretches even during exercise, is less likely to cause inappropriate compression, and is preferable for inner wear and sports underwear. To stably obtain the above compression, the above bare-yarn plain knitting in tubular knitting and the half structure in warp knitting are particularly preferable.

The measurement device 102 used for the biosignal detecting garments 100 and 100A of the present invention is preferably attached and connected to and detached from the garment body 104 via a connector. Detaching the measurement device 102 from the garment enables washing. The connector is not limited to particular parts, and a socket commonly used for connecting cords may be used, for example. It is more preferable to use a plurality of metal snap fasteners that can fix the measurement device 102 to the garment body 104 at the same time.

The measurement device 102 preferably has a function of transferring data through communication with a mobile terminal or a personal computer. This function enables the data to be easily acquired, stored, and analyzed in the personal computer, for example. The measurement device 102 particularly preferably communicates with a mobile terminal or a personal computer through wireless communication. Wireless communication eliminates the need for causing a user to devote to the communication.

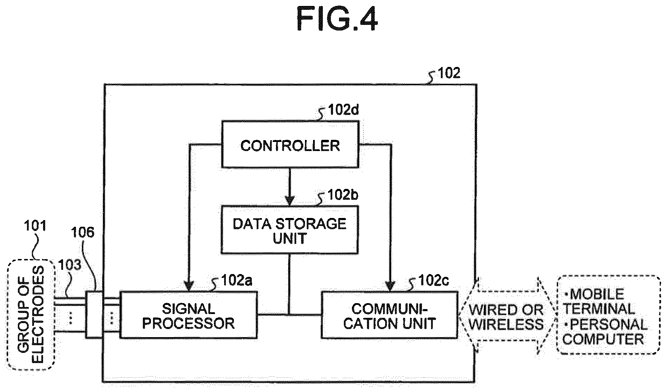

FIG. 4 is a schematic block diagram of the measurement device 102 used in the biosignal detecting garments 100 and 100A according to the embodiment of the present invention. As illustrated in FIG. 4, the measurement device 102 includes a signal processor 102a configured to process biosignals measured by the electrodes 101, a data storage unit 102b configured to store biosignal data processed by the signal processor 102a, a communication unit 102c configured to communicate the biosignal data to a mobile terminal or a personal computer through wired or wireless communication, and a controller 102d configured to control these units. The wiring portions 103 and the measurement device 102 that transmit biosignals are connected via a connector 106. This constitution of the measurement device 102 enables the data to be easily acquired, stored, and analyzed in the personal computer.

As described above, the biosignal detecting garment according to the present invention enables detection of biosignals, particularly cardiographic signals, with the form of clothes and enables continuous measurement of electrocardiograms and other signals over a long period of time without hindering activities of daily living. Examples

Next, the biosignal detecting garment of the present invention will be described in detail with reference to examples, but the biosignal detecting garment of the present invention is not limited to these examples.

(1) Load for Stretching with Elongation

The load for stretching with elongations of cloth used for the garment body was measured by applying JIS L1096A method. Specifically, three 5.0 cm width × 15 cm length test pieces were picked in the length or width direction and each elongated to an elongation percentage of 80% using a constant-rate-of-extension tensile tester with an autographic recording device by a cut strip method with a length of specimen between grips (between original marks) of 7.6 cm, an initial tension of 29 mN, and a tensile speed of 20 cm/min. Stress-strain curves were drawn, stresses at a distortion rate of 60%, in other words, loads for stretching were determined, average values were each calculated and rounded to one decimal place.

(2) Fineness

For sea-island composite fibers, a fabric was immersed in a 3% by mass aqueous solution of sodium hydroxide (75°C, with a bath ratio of 1:30) to dissolve and remove equal to or more than 99% of easily soluble components. Threads were then disassembled to select a multifilament constituted of ultrafine fiber filaments, and the mass of 1 m of the multifilament was measured. The fineness was calculated by multiplying the mass by 10,000. The procedure was repeated 10 times, and the fineness was defined as the value obtained by rounding the simple average to the first decimal place.

For other fibers, threads were disassembled to select a multifilament, and the mass of 1 m of the multifilament was measured. The fineness was calculated by multiplying the mass by 10,000. The procedure was repeated 10 times, and the fineness was defined as the value obtained by rounding the simple average to the first decimal place.

(3) Fiber Diameter

The obtained multifilament was embedded in an epoxy resin, frozen with an FC-4E cryosectioning system manufactured by Reichert, Inc., and cut with Reichert-Nissei Ultracut N (an ultramicrotome) equipped with a diamond knife. The cut surface was photographed with a VE-7800 scanning electron microscope (SEM) manufactured by KEYENCE Corp. at a magnification of 5,000 for nanofibers, 1,000 for microfibers, and 500 for the others. From the photographs obtained, 150 ultrafine fiber filaments randomly selected were sampled, and every circumscribed circle diameter (fiber diameter) was measured using image processing software (WinROOF) on the photographs.

(4) Fiber Diameter and Variation of Fiber Diameter (CV% (A)) of Multifilament

The average fiber diameter and the standard deviation of the fiber diameters of the above fiber diameters were calculated, and a fiber diameter CV% (coefficient of variation) was calculated on the basis of the equation below. All the above values are obtained by performing measurements for each photograph of 3 sites, obtaining the average value of the 3 sites, performing measurements in units of nanometers to one decimal place, and rounding the values to the whole number.

$$\text{The variation of fiber diameter (CV\% (A))} = \text{(the standard deviation of fiber diameters/the average fiber diameter)} \times 100$$

(5) Modification Ratio and Variation of Modification Ratios (CV% (B))

By a method similar to the above method for fiber diameters, the cross-section of the multifilament was photographed, and a circumscribed circle diameter (the fiber diameter) was defined as the diameter of a perfect circle circumscribing the cut plane on the basis of the image. In addition, an inscribed circle diameter was defined as the diameter of an inscribed perfect circle, and a value was calculated to three decimal places by the modification ratio = the circumscribed circle diameter ÷ the inscribed circle diameter. The value was rounded to two decimal places to obtain the modification ratio. The modification ratios were measured for 150 ultrafine fiber filaments randomly sampled in the same image, and a variation of modification ratios (CV% (B) (coefficient of variation)) was calculated from the average value and the standard deviation on the basis of the equation below.
This variation of modification ratios is rounded to one decimal place.

$$\text{The variation of modification ratios (CV\% (B))} = \text{(the standard deviation of modification ratios/the average value of modification ratios)} \times 100 \text{ (\%)}$$

(6) Quantity of Attached Resin

A quantity of the attached resin was measured on the basis of changes in the mass of a fiber structure that was a test fabric between before and after applying a dispersion of a conductive polymer in the standard state (20°C × 65% RH). The calculation expression is as follows.

$$\text{The quantity of the attached resin (g/m}^2\text{)} = \text{(the mass of the test fabric after being treated (g)} - \text{the mass or the weight of the test before being treated (g))/the area of the test fabric on which the dispersion is applied (m}^2\text{)}$$

(7) Surface Resistance

An electrode of 10 cm × 10 cm was used as a test piece and placed on high-quality expanded polystyrene. A surface resistance value ($\Omega$) was measured with a resistance meter (Loresta-AX MCP-T370, a 4-probe resistance meter manufactured by Mitsubishi Chemical Analytech Co., Ltd.) under an environment of 20°C and 40% RH.

(8) Washing Durability

An electrode of 10 cm × 10 cm was used as a test piece, and a surface resistance was measured after washing by a 20-time repeating method by a method in accordance with JIS L0217 (2012) 103 method. An automatic washing machine (National NA-F50Z8) was used as the washing machine.

(9) Breathability

The breathability of an electrode was measured in accordance with the air permeability A method (the Frazier method) in JIS L1096 (testing methods for woven and knitted fabrics) (1999) .

(10) Bending Resistance

The bending resistance of the electrode was measured in accordance with the bending resistance A method (the 45° cantilever method) in JIS L1096 (testing methods for woven and knitted fabrics) (1999).

Production examples and an example of the electrodes, the garment body, the wiring portions, and the electric insulating members used in the biosignal detecting garment of the present invention will be described. Production Examples of Electrodes

[Production Example 1]

A tubular knitted fabric was knitted into an interlock structure using a polyester-nanofiber combined-filament yarn of 100T-136F obtained by combining a high-shrinkage yarn of 22T-24F with a nanofiber of 75T-112F (with a composite ratio of the sea/island components of 30%:70%, the number of the islands of 127/F) of an alkaline-hot-water soluble polyester in which the island component was polyethylene terephthalate and the sea component was a polyester copolymer containing terephthalic acid and sodium 5-sulfoisophthalate as the acid component. Next, the fabric was immersed in a 3% by mass aqueous solution of sodium hydroxide (75°C, with a bath ratio of 1:30) to remove easily soluble components. A knitted fabric was obtained using the combined-filament yarn of the nanofiber and the high-shrinkage yarn. To the knitted fabric obtained as a fiber structure, a dispersion in which 1.0% by weight of PEDOT/PSS as a conductive polymer and 5.0% by weight of an acrylic thermosetting resin as a binder were dispersed in a mixed solvent of water and ethanol (44% by weight of water and 50% by weight of ethanol) was applied by a known gravure coating method so that the quantity of the applied agent would be 15 g/m$^2$ to obtain an electrode. Table 1 and Table 2 list the materials used and properties of the obtained electrode.

[Production Example 2]

An electrode was produced through the same treatments as those for Production Example 1 except that the high-shrinkage yarn of 22T-246F was changed to a high-shrinkage yarn of 33T-6F and a polyester-nanofiber combined-filament yarn of 110T-118F obtained by crossing the high-shrinkage yarn with the nanofiber of 75T-112F (with a composite ratio of the sea/island components of 30%:70%, the number of the islands of 127/F) was used. Table 1 and Table 2 list the materials used and properties of the obtained electrode.

[Production Example 3]

An electrode was produced through the same treatments as those for Production Example 1 except that the fabric structure was changed from knitted fabric to plain weave fabric. Table 1 lists properties of the materials used. Table 1 and Table 2 list the materials used and properties of the obtained electrode.

[Production Example 4]

An electrode was produced through the same treatments as those for Production Example 1 except that the high-shrinkage yarn of 22T-24F was not used and the yarn was changed to a polyester-nanofiber single yarn of 75T-112F (with a composite ratio of the sea/island components of 30%:70%, the number of the islands of 127/F). Table 1 and Table 2 list the materials used and properties of the obtained electrode.

[Production Example 5]

An electrode was produced through the same treatments as those for Production Example 1 except that the high-shrinkage yarn of 22T-24F was not used and the yarn of 75T-112F (with a composite ratio of the sea/island components of 30%:70%, the number of the islands of 127/F) was changed to a polyester-nanofiber single yarn of 100T-30F (with a composite ratio of the sea/island components of 30%:70%, the number of the islands of 2048/F). Table 1 and Table 2 list the materials used and properties of the obtained electrode.

[Production Example 6]

An electrode was produced through the same treatments as those for Production Example 1 except that the high-shrinkage yarn of 22T-24F was not used and the yarn of 75T-112F (with a composite ratio of the sea/island components of 30%:70%, the number of the islands of 127/F) was changed to polyester-nanofiber single yarn of 120T-60F (with a composite ratio of the sea/island components of 50%:50%, the number of the islands of 2048/F). Table 1 lists the materials used and properties of the obtained electrode.

[Production Example 7]

An electrode was produced through the same treatments as those for Production Example 1 except that the high-shrinkage yarn of 22T-24F was not used and the yarn was changed to a polyester-nanofiber single yarn of 75T-112F (with a composite ratio of the sea/island components of 30%:70%, the number of the islands of 127/F) having a triangular cross-section. Table 1 and Table 2 list the materials used and properties of the obtained electrode.

[Production Example 8]

An electrode was produced through the same treatments as those for Production Example 1 except that the high-shrinkage yarn of 22T-24F was not used and the fabric was changed from a fabric of 75T-112F (with a composite ratio of the sea/island components of 30%:70%, the number of the islands of 127/F) to a woven fabric of a microfiber of 66T-9F (with a composite ratio of the sea/island components of 20%:80%, the number of the islands of 70/F). Table 1 and Table 2 list the materials used and properties of the obtained electrode.

[Production Example 9]

A polyurethane was added by impregnation to a needle-punched nonwoven fabric having been formed using a polymer array fiber (with a composite ratio of the sea/island components of 57%:43%, the number of the islands of 16) of 4.2 dtex having a length of 51 mm in which the island component was a polyethylene terephthalate and the sea component was a polystyrene, and wet-solidifying was performed. The content of the polyurethane was 49% of the mass of polyethylene terephthalate. The product was immersed in trichloroethylene and squeezed with a mangle to remove the polystyrene component. An ultrafine fiber having a single-yarn fineness of 0.15 dtex was obtained. A nonwoven fabric having been subjected to nap raising with a buffing machine and dyeing was obtained. In the same manner as in Production Example 1, a dispersion in which PEDOT/PSS as a conductive polymer and an acrylic thermosetting resin as a binder were dispersed in a mixed solvent of water and ethanol was then applied to the nonwoven fabric obtained as a fiber structure by a known gravure coating method so that the quantity of the applied agent would be 15 g/m$^2$ to obtain an electrode. Table 1 and Table 2 list the materials used and properties of the obtained electrode.

[Production Example 10]

An electrode was produced through the same treatments as those for Production Example 1 except that the high-shrinkage yarn of 22T-24F was not used and the fabric was changed from a fabric of 75T-112F (with a composite ratio of the sea/island components of 30%:70%, the number of the islands of 127/F) to a woven fabric of a polyester fiber of 84T-36F (a polyester fiber cloth for dyeing tests manufactured by Shikisensha Co., Ltd.). Table 1 and Table 2 list the materials used and properties of the obtained electrode.

[Production Example 11]

A tubular knitted fabric was knitted using a combined-filament yarn obtained by combining a polyester fiber of 56T-24F with a polyurethane yarn. Next, the fabric was immersed in a mixed aqueous solution of 0.06% by mass of sodium hydroxide and 0.05% by mass of a surfactant (80°C, with a bath ratio of 1:30) to remove oil agents in original yarn and dirt. In the same manner as in Production Example 1, a dispersion of a conductive polymer was applied to the knitted fabric obtained as a fiber structure to obtain an electrode. Table 1 and Table 2 list the materials used and properties of the obtained electrode.

[Production Example 12]

A tubular knitted fabric was knitted using a nylon-fiber single yarn of 78T-24F. Next, the fabric was immersed in a mixed aqueous solution of 0.06% by mass of sodium hydroxide and 0.05% by mass of a surfactant (80°C, with a bath ratio of 1:30) to remove oil agents in original yarn and dirt. In the same manner as in Production Example 1, a dispersion of a conductive polymer was applied to the knitted fabric obtained as a fiber structure to obtain an electrode. Table 1 and Table 2 list the materials used and properties of the obtained electrode.

[Production Example 13]

A tubular knitted fabric was knitted using a polyester-nanofiber combined-filament yarn of 100T-136F obtained by combining a high-shrinkage yarn of 22T-24F with a nanofiber of 75T-112F (with a composite ratio of the sea/island components of 30%:70%, the number of the islands of 127/F) of an alkaline-hot-water soluble polyester in which the island component was a polyethylene terephthalate and the sea component was a polyester copolymer containing terephthalic acid and sodium 5-sulfoisophthalate as the acid component. Next, the fabric was immersed in a 3% by mass aqueous solution of sodium hydroxide (75°C, with a bath ratio of 1:30) to remove easily soluble components. A knitted fabric was obtained using the combined-filament yarn of the nanofiber and the high-shrinkage yarn. A polyurethane-resin microporous membrane was laminated on the back face of the obtained knitted fabric by a known method. A dispersion in which PEDOT/PSS as a conductive polymer and an acrylic thermosetting resin as a binder were dispersed in a mixed solvent of water and ethanol was applied to the front face by a known gravure coating method so that the quantity of the applied agent would be 15 g/m$^2$ to obtain an electrode. Table 1 and Table 2 list the materials used and properties of the obtained electrode.

[Production Example 14]

An electrode was produced through the same treatments as those for Production Example 13 except that the high-shrinkage yarn of 22T-24F was changed to a high-shrinkage yarn of 33T-6F and the yarn was changed to a polyester-nanofiber combined-filament yarn of 110T-118F including the nanofiber of 75T-112F (with a composite ratio of the sea/island components of 30%:70%, the number of the islands of 127/F). Table 1 and Table 2 list the materials used and properties of the obtained electrode.

[Production Example 15]

An electrode was obtained through the same treatments as those for Production Example 13 except that the fabric structure was changed from knitted fabric to plain weave fabric. Table 1 and Table 2 list the materials used and properties of the obtained electrode.

[Production Example 16]

An electrode was obtained through the same treatments as those for Production Example 13 except that the yarn was changed to a polyester-nanofiber single yarn of 75T-112F (with a composite ratio of the sea/island components of 30%:70%, the number of the islands of 127/F). Table 1 and Table 2 list the materials used and properties of the obtained electrode.

[Production Example 17]

A polyurethane was added by impregnation to a needle-punched nonwoven fabric having been formed using a polymer array fiber (with a composite ratio of the sea/island components of 57%:43%, the number of the islands of 16) of 4.2 dtex and 51 mm in which the island component was a polyethylene terephthalate and the sea component was a polystyrene, and wet-solidifying was performed. The content of the polyurethane was 49% of the mass of polyethylene terephthalate. The product was immersed in trichloroethylene and squeezed with a mangle to remove the polystyrene component. An ultrafine fiber having a single-yarn fineness of 0.15 dtex was obtained. A nonwoven fabric having been subjected to nap raising with a buffing machine and dyeing was obtained. In the same manner as in Production Example 13, a polyurethane-resin microporous membrane was laminated on the back face of the obtained nonwoven fabric, and a dispersion of a conductive polymer was applied to the front face to obtain an electrode. Table 1 and Table 2 list the materials used and properties of the obtained electrode.

[Production Example 18]

Using a polyester-fiber woven fabric of 84T-36F (a polyester fiber cloth for dyeing tests manufactured by Shikisensha Co., Ltd.), a polyurethane-resin microporous membrane was laminated on the back face of the fabric, and a dispersion of a conductive polymer was applied to the front face to obtain an electrode in the same manner as in Production Example 13. Table 1 and Table 2 list the materials used and properties of the obtained electrode.

[Production Example 19]

A tubular knitted fabric was knitted using a combined-filament yarn obtained by combining a polyester fiber of 56T-24F with a polyurethane yarn. Next, the fabric was immersed in a mixed aqueous solution of 0.06% by mass of sodium hydroxide and 0.05% by mass of a surfactant (80°C, with a bath ratio of 1:30) to remove oil agents in original yarn and dirt. In the same manner as in Production Example 13, a polyurethane-resin microporous membrane was laminated on the back face of the obtained knitted fabric, and a dispersion of a conductive polymer was applied to the front face to obtain an electrode. Table 1 and Table 2 list the materials used and properties of the obtained electrode.

[Production Example 20]

A tubular knitted fabric was knitted using a nylon-fiber single yarn of 78T-24F. Next, the fabric was immersed in a mixed aqueous solution of 0.06% by mass of sodium hydroxide and 0.05% by mass of a surfactant (80°C, with a bath ratio of 1:30) to remove oil agents in original yarn and dirt. A polyurethane-resin microporous membrane was laminated on the back face of the obtained knitted fabric, and a dispersion of a conductive polymer was applied to the front face to obtain an electrode. Table 1 and Table 2 list the materials used and properties of the obtained electrode.

[Production Example 21]

An electrode was obtained through the same treatments as those for Production Example 1 except that the conductive polymer was changed to a 5% polyaniline aqueous solution (manufactured by Sigma-Aldrich Co. LLC.). Table 1 and Table 2 list the materials used and properties of the obtained electrode.

[Production Example 22]

An electrode was obtained through the same treatments as those for Production Example 1 except that the conductive polymer was changed to a 5% polypyrrole aqueous solution (manufactured by Sigma-Aldrich Co. LLC.). Table 1 and Table 2 list the materials used and properties of the obtained electrode.

[Production Example 23]

An electrode was obtained through the same treatments as those for Production Example 1 except that the polyester nanofiber of Production Example 4 was changed to a nylon nanofiber. Table 1 and Table 2 list the materials used and properties of the obtained electrode.

Production Examples of Body Fabric for Garment Body

[Production Example 24]

A gray fabric of a half structure was produced with a tricot machine using polyester filaments of 56T-24F at the front reed and a polyurethane elastic fiber of 33T at the back reed. Scouring and relaxation were then performed. Thermal setting at 190°C was performed prior to dyeing under ordinary dyeing conditions for polyesters, and finishing setting at 170°C was performed to obtain a body fabric of 180 g/m². The stress at an elongation of 60% in the breadth direction was 5.2 N.

[Production Example 25]

A gray fabric of a bare-yarn plain structure was produced with a 32-gauge tubular knitting machine with paralleled yarn of polyester false-twisted yarn of 84T-36F and polyurethane elastic yarn of 33T. Scouring and relaxation were then performed. Thermal setting at 185°C was performed prior to dyeing under ordinary dyeing conditions for polyesters, and final finishing setting at 170°C was performed to obtain a body fabric having an areal weight of 180 g/m². The stress at an elongation of 60% in the breadth direction was 2.1 N.

Production Examples of Wiring Portions

[Production Example 26]

As wiring portions, a mixed resin of a carbon resin "LIONPASTE W-311" manufactured by Lion Corporation and an acrylic resin "Stretch Clear 701B" manufactured by MATSUI SHIKISO CHEMICAL Co., Ltd. in the ratio of 1:4 was printed in a form of wiring on the surfaces of garment bodies produced from the body fabrics produced in Production Examples 24 and 25 in a thickness of 20 μm by screen printing.

[Production Example 27]

Sewing was performed using paralleled and twisted thread of two threads of 110T-34F of silver-coated thread "AGposs" manufactured by Mitsufuji Textile Ind. Co., Ltd. as a conductive fiber as the bobbin thread and using No. 60 count polyester sewing thread as the needle thread with the inner faces of garment bodies produced from the body fabrics produced in Production Examples 24 and 25 facing upward. The silver-coated thread was thus sewn on the surfaces of the cloth.

Production Examples of Waterproof Electric Insulating Members

[Production Example 28]

As waterproof electric insulating members for covering the wiring portions produced in Production Examples 26 and 27, waterproof polyurethane seam tape "αE-110" manufactured by Toray Coatex Co., Ltd. was used to cover the wiring portions exposed on the garment bodies.

[Production Example 29]

As waterproof electric insulating members, a polytetrafluoroethylene (PTFE) film on one face of which a polyurethane hot-melt adhesive has been applied was cut into tape-shaped pieces to produce waterproof seam tape. The wiring portions produced in Production Examples 26 and 27 were covered with the produced waterproof seam tape.

Example 1

A biosignal detecting garment was produced by combining the body fabric produced in Production Example 24 with the electrodes of Production Example 2, the wires of Production Example 27, and the electric insulating members of Production Example 28 according to the following specifications. Metal snap fasteners for clothing manufactured by YKK Inc. were used as a tool for attaching the measurement device to the garment body.

As illustrated in FIG. 1, in the biosignal detecting garment produced in Example 1, the electrodes 101a and 101b were placed at about positions on the right and left fifth ribs 2 in the armpits, and the measurement device 102 that was an electrocardiogram measurement device acquired electrocardiographic waveforms using the electrode 101b placed in the left armpit as a positive different electrode (the positive pole) and the electrode 101a placed in the right armpit as a negative different electrode (the negative pole), so that electrocardiographic waveforms similar to those in the CC5 lead acquired by conventional Holter monitors were able to be detected. Waveforms in the CC5 lead are advantageous to automatic analysis of pulse intervals (R-R intervals) and the like because conventional findings in clinical medicine can be applied and detection can be performed so that the amplitudes of QRS signals will be large.

The electrode 101c placed at a position separated from the electrodes 101a and 101b placed at about the right and left sides of the chest or the flank may be freely placed as long as the electrode has contact with the body 1. Placing at least part of the electrode 101c on the right or left scapula region or costal arch 3 particularly makes it easy for the electrode 101c to have contact with the body 1 and stabilizes detection of biosignals.

Providing the measurement device 102 on the right or left chest, shoulder, or lumber region has the effects that mixing of noise due to movements of a user is prevented, that effects on activities of daily living of the user are small, and that the user can easily put on and remove the terminal. It is preferable that the user can make minor adjustments of the attachment position of the measurement device 102 by him- or herself using, for example, a conductive hook and loop fastener as a connector because burdens on the user can be further reduced.

As illustrated in FIG. 4, for example, the measurement device 102 used included the signal processor 102a configured to receive signals from the electrodes 101 and detect electrocardiograms, the data storage unit 102b configured to store the received signals, the detected electrocardiogram data, and the like, the communication unit 102c configured to communicate and exchange data with a mobile terminal or a personal computer, and the controller 102d configured to control these functional blocks.

Table 1

| | Filament | Polymer | Cross-section | Fineness | Fiber diameter | Use of yarn | Variation of fiber diameter (CV% (A)) | Variation of modification ratio (CV% (B))) | Density (yarns /in) length × breadth | Areal weight (g/cm$^2$) | Fiber structure | Conductive polymer | quantity of applied resin (g/cm$^2$) |
|---|---|---|---|---|---|---|---|---|---|---|---|---|---|
| Production Example 1 | Multifilament /High-shrinkage yarn | Polyester | Round | 0.004 dtex /0.9 dtex | 700 nm | 75T-112F (island component 30%:70%) /22T-24F | 5 | 7 | 58 × 78 | 118 | Knitted fabric | PEDOT/PSS | 14.3 |
| Production Example 2 | Multifilament /High-shrinkage yarn | Polyester | Round | 0.004 dtex /5.5 dtex | 700 nm | 75T-112F (island component 30%:70%) /33T-6F | 5 | 7 | 46 × 110 | 194 | Knitted fabric | PEDOT/PSS | 14.5 |
| Production Example 3 | Multifilament /High-shrinkage yarn | Polyester | Round | 0.004 dtex /0.9 dtex | 700 nm | 75T-112F (island component 30%:70%) /22T-24F | 5 | 7 | 216 × 113 | 98 | Woven fabric | PEDOT/PSS | 11.2 |
| Production Example 4 | Multifilament | Polyester | Round | 0.004 dtex | 700 nm | 75T-112F (island component 30%:70%) | 5 | 7 | 43 × 58 | 112 | Knitted fabric | PEDOT/PSS | 13.2 |
| Production Example 5 | Multifilament | Polyester | Round | 0.001 dtex | 300 nm | 100T-30F (island component 30%:70%) | 3 | 3.4 | 58 × 78 | 110 | Knitted fabric | PEDOT/PSS | 14.8 |
| Production Example 6 | Multifilament | Polyester | Round | 0.0004 dtex | 200 nm | 120T-60F (island component 50%:50%) | 3 | 3.4 | 70 × 94 | 98 | Knitted fabric | PEDOT/PSS | 15.3 |

(continued)

| | Filament | Polymer | Cross-section | Fineness | Fiber diameter | Use of yarn | Variation of fiber diameter (CV% (A)) | Variation of modification ratio (CV% (B)) | Density (yarns/in) length × breadth | Areal weight (g/cm²) | Fiber structure | Conductive polymer | quantity of applied resin (g/cm²) |
|---|---|---|---|---|---|---|---|---|---|---|---|---|---|
| Production Example 7 | Multifilament | Polyester | triangular | 0.004 dtex | 700 nm | 75T-112F (island component 30%:70%) | 3 | 3.4 | 43 × 58 | 115 | Knitted fabric | PEDOT/PSS | 13.0 |
| Production Example 8 | Multifilament | Polyester | Round | 0.07 dtex | 2700 nm | 66T-9F (island component 20%:80%) | 6 | 9 | 114 × 118 | 61 | Woven fabric | PEDOT/PSS | 10.2 |
| Production Example 9 | Multifilament | Polyester | Round | 0.15 dtex | 3800 nm | 0.15 dtex single-yarn fineness | 6 | 9 | - | 135 | Nonwoven fabric | PEDOT/PSS | 15.2 |
| Production Example 10 | Multifilament | Polyester | Round | 2.3 dtex | 15000 nm | 84T-36F | 4 | 4.2 | 105 × 95 | 68 | Woven fabric | PEDOT/PSS | 12.8 |
| Production Example 11 | Multifilament | Polyester /Polyurethane | Round | 2.3 dtex | 15000 nm | 56T-24F /22T (PU) | 4 | 4.2 | 67 × 62 | 176 | Knitted fabric | PEDOT/PSS | 13.9 |
| Production Example 12 | Multifilament | Nylon | Round | 3.3 dtex | 36000 nm | 78T-24F | 3.5 | 3.7 | 32 × 40 | 88 | Knitted fabric | PEDOT/PSS | 13.2 |
| Production Example 13 | Multifilament /High-shrinkage yarn | Polyester | Round | 0.004 dtex /0.9 dtex | 700 nm | 75T-112F (island component 30%:70%) /22T-24F | 5 | 7 | 58 × 78 | 118 | Knitted fabric | PEDOT/PSS | 15.5 |
| Production Example 14 | Multifilament /High-shrinkage yarn | Polyester | Round | 0.004 dtex /5.5 dtex | 700 nm | 75T-112F (island component 30%:70%) /33T-6F | 5 | 7 | 46 × 110 | 194 | Knitted fabric | PEDOT/PSS | 15.3 |

| | Filament | Polymer | Cross-section | Fineness | Fiber diameter | Use of yarn | Variation of fiber diameter (CV% (A)) | Variation of modification ratio (CV% (B)) | Density (yarns /in) length × breadth | Areal weight (g/cm²) | Fiber structure | Conductive polymer | quantity of applied resin (g/cm²) |
|---|---|---|---|---|---|---|---|---|---|---|---|---|---|
| Production Example 15 | Multifilament /High-shrinkage yarn | Polyester | Round | 0.004 dtex /0.9 dtex | 700 nm | 75T-112F (island component 30%:70%) /22T-24F | 5 | 7 | 216 × 113 | 98 | Woven fabric | PEDOT/PSS | 11.7 |
| Production Example 16 | Multifilament | Polyester | Round | 0.004 dtex | 700 nm | 75T-112F (island component 30%:70%) | 5 | 7 | 43 × 58 | 112 | Knitted fabric | PEDOT/PSS | 15.8 |
| Production Example 17 | Multifilament | Polyester | Round | 0.15 dtex | 3800 nm | 0.15 dtex single-yarn fineness | 6 | 9 | - | 135 | Nonwoven fabric | PEDOT/PSS | 13.3 |
| Production Example 18 | Multifilament | Polyester | Round | 2.3 dtex | 15000 nm | 84T-36F | 4 | 4.2 | 105 × 95 | 68 | Woven fabric | PEDOT/PSS | 12.2 |
| Production Example 19 | Multifilament | Polyester /Polyurethane | Round | 2.3 dtex | 15000 nm | 56T-24F /22T(PU) | 4 | 4.2 | 67 × 62 | 176 | Knitted fabric | PEDOT/PSS | 15.0 |
| Production Example 20 | Multifilament | Nylon | Round | 3.3 dtex | 36000 nm | 78T-24F | 3.5 | 3.7 | 32 × 40 | 88 | Knitted fabric | PEDOT/PSS | 15.6 |
| Production Example 21 | Multifilament /High-shrinkage yarn | Polyester | Round | 0.004 dtex /0.9 dtex | 700 nm | 75T-112F (island component 30%:70%) /22T-24F | 5 | 7 | 58 × 78 | 118 | Knitted fabric | Polyaniline | 15.2 |
| Production Example 22 | Multifilament /High-shrinkage yarn | Polyester | Round | 0.004 dtex /0.9 dtex | 700 nm | 75T-112F (island component 30%:70%) /22T-24F | 5 | 7 | 58 × 78 | 118 | Knitted fabric | Polypyrrole | 14.8 |

EP 3 100 677 A1

(continued)

| | Filament | Polymer | Cross-section | Fineness | Fiber diameter | Use of yarn | Variation of fiber diameter (CV% (A)) | Variation of modification ratio (CV% (B))) | Density (yarns /in) length × breadth | Areal weight (g/cm$^2$) | Fiber struc-ture | Conductive polymer | quantity of ap-plied res-in (g/cm$^2$) |
|---|---|---|---|---|---|---|---|---|---|---|---|---|---|
| Production Example 23 | Multifilament | Nylon | Round | 0.004 dtex | 700 nm | 75T-112F (island component 30%:70%) | 5 | 7 | 45 × 60 | 115 | Knitted fabric | PEDOT/PSS | 13.5 |

Table 2

| | moisture-permeable layer | dyeing | Chemical treatment | Physical treatment | Resistance ($\Omega$) | Resistance (washing) | Breathability (cc/cm$^2$/sec) | Bending resistance length × breadth |
|---|---|---|---|---|---|---|---|---|
| Production Example 1 | - | - | - | - | 57.7 | $1.1 \times 10^5$ | 150 | 15 × 16 |
| Production Example 2 | - | - | - | - | 63.1 | $0.42 \times 10^5$ | 180 | 22 × 25 |
| Production Example 3 | - | - | - | - | 36.5 | $1.4\,4 \times 104$ | 0.521 | 47 × 38 |
| Production Example 4 | - | - | - | - | 60.3 | $2.8 \times 10^5$ | 140 | 12 × 14 |
| Production Example 5 | - | - | - | - | 35.2 | $1.8 \times 104$ | 130 | 10 × 11 |
| Production Example 6 | - | - | - | - | 25.5 | $2.5 \times 10^4$ | 126 | 10 × 12 |
| Production Example 7 | - | - | - | - | 64.5 | $2.4 \times 10^5$ | 135 | 15 × 16 |
| Production Example 8 | - | - | - | - | 29.3 | Equal to or more than $10^6$ | 43 | 39 × 27 |
| Production Example 9 | - | - | PU | Nap raising | 37.2 | $0.41 \times 10^4$ | 10.4 | 42 × 43 |
| Production Example 10 | - | - | - | - | 21.5 | Equal to or more than $10^6$ | Equal to or more than 600 | 49 × 43 |
| Production Example 11 | - | - | - | - | 16.5 | $0.32 \times 10^5$ | 250 | 25 × 33 |
| Production Example 12 | - | - | - | - | 22.1 | $0.98 \times 10^5$ | 401 | 37 × 46 |
| Production Example 13 | existence | - | - | - | 15.3 | $0.22 \times 10^3$ | 0 | 32 × 33 |
| Production Example 14 | existence | - | - | - | 19.3 | $0.28 \times 10^3$ | 0 | 38 × 40 |

| | moisture-permeable layer | dyeing | Chemical treatment | Physical treatment | Resistance ($\Omega$) | Resistance (washing) | Breathability (cc/cm$^2$/sec) | Bending resistance length $\times$ breadth |
|---|---|---|---|---|---|---|---|---|
| Production Example 15 | existence | - | - | - | 30.3 | $0.40 \times 10^3$ | 0 | $69 \times 59$ |
| Production Example 16 | existence | - | - | - | 16.8 | $1.4 \times 10^3$ | 0 | $25 \times 33$ |
| Production Example 17 | existence | dyeing | PU | Nap raising | 38.1 | $0.57 \times 10^3$ | 0 | $42 \times 43$ |
| Production Example 18 | existence | - | - | - | 21.3 | Equal to or more than $10^6$ | 0 | $69 \times 57$ |
| Production Example 19 | existence | - | - | - | 16.6 | $0.4 \times 104$ | 0 | $17 \times 23$ |
| Production Example 20 | existence | - | - | - | 16.1 | $0.29 \times 10^4$ | 0 | $36 \times 52$ |
| Production Example 21 | - | - | - | - | 43.2 | $6.8 \times 10^5$ | 160 | $18 \times 19$ |
| Production Example 22 | - | - | - | - | 50.8 | $7.2 \times 10^5$ | 165 | $20 \times 21$ |
| Production Example 23 | - | - | - | - | 40.3 | $1.4 \times 104$ | 138 | $25 \times 33$ |

Reference Signs List

1    body

| | |
|---|---|
| 2 | fifth ribs |
| 3 | scapula region or costal arch |
| 100, 100A | biosignal detecting garments |
| 101 | electrodes |
| 102 | measurement device |
| 103 | wiring portions |
| 104 | garment body |
| 105 | electric insulating members |
| 106 | connector |
| 110 | conductive connection systems |
| 120 | water-repellent and insulating structures |

**Claims**

1.  A biosignal detecting garment comprising:

    at least two or more electrodes each including a conductive fiber structure;
    a measurement device configured to detect and process a bioelectric signal acquired by the electrodes in contact with a living body;
    a wiring portion conductively connecting the electrodes to the measurement device; and
    a garment body on which the electrodes, the measurement device, and the wiring portion are placed at predetermined positions.

2.  The biosignal detecting garment according to claim 1, wherein
    the measurement device is an electrocardiogram measurement device,
    any one of the electrodes is used as a different electrode,
    at least one electrodes other than the different electrode is used as at least one indifferent electrode (reference biopotential electrode), and
    a potential difference between the different electrode and the indifferent electrode is detected as an electrocardiographic waveform.

3.  The biosignal detecting garment according to claim 1, wherein
    the measurement device is an electrocardiogram measurement device,
    the number of the electrodes each including the conductive fiber structure is at least three or more,
    any two of the electrodes are used as different electrodes,
    at least one electrode other than the different electrodes is used as at least one indifferent electrode (reference biopotential electrode), and
    a potential difference between the two different electrodes is detected as an electrocardiographic waveform.

4.  The biosignal detecting garment according to any one of claims 1 to 3, wherein
    the measurement device is an electrocardiogram measurement device,
    two of the electrodes are respectively placed at about right and left sides of a chest or a flank of the garment body, and
    when three or more of the electrodes are included, rest of the electrodes is placed at a position separated from the electrodes placed at about the right and left sides of the chest or the flank of the garment body.

5.  The biosignal detecting garment according to claim 4, wherein
    the electrodes respectively placed at about the right and left sides of the chest or the flank of the garment body are used as two different electrodes,
    the rest of the electrode is used as at least one indifferent electrode (reference biopotential electrode), and
    a potential difference between the two different electrodes is detected as an electrocardiographic waveform.

**6.** The biosignal detecting garment according to any one of claims 1 to 5, wherein the conductive fiber structure is a fiber structure impregnated with a conductive polymer.

**7.** The biosignal detecting garment according to claim 6, wherein a dispersion in which the conductive polymer and a binder are dispersed in a solvent is applied to the conductive fiber structure to impregnate the fiber structure with the conductive polymer.

**8.** The biosignal detecting garment according to claim 6 or 7, wherein the conductive polymer is a mixture of a poly(3,4-ethylenedioxythiophene) and a polystyrenesulfonic acid.

**9.** The biosignal detecting garment according to any one of claims 1 to 8, a fiber structure used for the electrodes includes a woven or knitted fabric having an areal weight of equal to or more than 50 $g/m^2$ and equal to or less than 300 $g/m^2$.

**10.** The biosignal detecting garment according to any one of claims 1 to 9, wherein a woven or knitted fabric used for the electrodes includes a synthetic fiber multifilament at least part of which has a fineness of equal to or more than 30 dtex and equal to or less than 400 dtex and a single-yarn fineness of equal to or less than 0.2 dtex.

**11.** The biosignal detecting garment according to any one of claims 1 to 10, wherein a woven or knitted fabric used for the electrodes includes a synthetic fiber multifilament at least part of which has a single-yarn diameter of equal to or more than 10 nm and equal to or less than 5,000 nm.

**12.** The biosignal detecting garment according to any one of claims 1 to 11, wherein a woven or knitted fabric used for the electrodes includes a synthetic fiber multifilament at least part of which has a single-yarn diameter of equal to or more than 10 nm and equal to or less than 1,000 nm.

**13.** The biosignal detecting garment according to any one of claims 1 to 12, further comprising a resin layer that is layered on a face of the conductive fiber structure used for the electrodes, the face being opposite to another face configured to have contact with skin.

**14.** The biosignal detecting garment according to claim 13, wherein the resin layer includes a polyurethane-based moisture-permeable layer.

**15.** The biosignal detecting garment according to any one of claims 1 to 14, wherein the wiring portion is formed of a printed conductive resin, a laminated conductive resin film, a conductive fiber, or a metal wire.

**16.** The biosignal detecting garment according to any one of claims 1 to 15, wherein the wiring portion is formed by sewing-in of a conductive fiber, the conductive fiber comprising a fiber coated with a metal.

**17.** The biosignal detecting garment according to claim 16, wherein the metal with which the conductive fiber is coated includes silver, aluminum, or stainless steel.

**18.** The biosignal detecting garment according to any one of claims 1 to 17, wherein the wiring portion is disposed on an outer side of the garment body.

**19.** The biosignal detecting garment according to any one of claims 1 to 18, wherein the wiring portion is formed by sewing-in of a conductive fiber, the conductive fiber being sewn in as one thread of a sewing machine by sewing so as to be exposed mainly on an outer side of the garment body.

**20.** The biosignal detecting garment according to any one of claims 1 to 19, wherein
the wiring portion is disposed on an outer side of the garment body, and
part of the wiring portion exposed on the outer side of the garment body is covered with a waterproof electric insulating member.

**21.** The biosignal detecting garment according to claim 20, wherein the electric insulating member includes a polyurethane-based film.

**22.** The biosignal detecting garment according to any one of claims 1 to 15, 18, and 21, wherein

the wiring portion is formed of a conductive resin,
and
the wiring portion is formed with the conductive resin being continuously layered on part of one face of a sheet including a waterproof electric insulating member, and with the face of the waterproof electric insulating member on which the conductive resin is layered being bonded to the garment body.

23. The biosignal detecting garment according to any one of claims 1 to 22, further comprising at least two conductive connection systems, the conductive connection systems each including:

> one of the electrodes;
> the measurement device; and
> the wiring portion conductively connecting the electrode to the measurement device, wherein
> at least parts of the conductive connection systems formed on the garment body are separated from each other by a water-repellent and insulating structure.

24. The biosignal detecting garment according to any one of claims 1 to 23, wherein
the garment body includes a woven or knitted fabric having a stress of equal to or more than 0.5 N and equal to or less than 15 N at an elongation of 60% in a length or breadth direction, and
the electrodes are closely attached to skin at a pressure of equal to or more than 0.1 kPa and equal to or less than 2.0 kPa when being worn.

25. The biosignal detecting garment according to any one of claims 1 to 24, wherein the garment body includes a woven or knitted fabric including elastic yarn and inelastic yarn.

26. The biosignal detecting garment according to claim 25, wherein the elastic yarn includes a polyurethane-based elastic fiber.

27. The biosignal detecting garment according to any one of claims 1 to 26, wherein the garment body includes a knitted fabric.

28. The biosignal detecting garment according to any one of claims 1 to 27, wherein the measurement device is configured to be attached and connected to and detached from the garment body via a connector.

29. The biosignal detecting garment according to any one of claims 1 to 28, wherein the measurement device has a function of transferring data through communication with at least one of a mobile terminal and a personal computer.

30. The biosignal detecting garment according to any one of claims 1 to 29, wherein the measurement device has a function of transferring data through wireless communication with at least one of a mobile terminal and a personal computer.

31. A biosignal detecting garment comprising:

> at least two or more electrodes each including a conductive fiber structure;
> a connector through which a measurement device configured to detect and process a bioelectric signal acquired by the electrodes that are in contact with a living body is configured to be attached, connected, and detached;
> a wiring portion conductively connecting the electrodes to the connector; and
> a garment body on which the electrodes, the connector, and the wiring portion are placed at predetermined positions.

# FIG.1

# FIG.2

# FIG.3

(a)

(b)

# FIG.4

**INTERNATIONAL SEARCH REPORT**

| International application No. |
| --- |
| PCT/JP2015/052229 |

A. CLASSIFICATION OF SUBJECT MATTER
*A61B5/0408*(2006.01)i, *A41D13/12*(2006.01)i

According to International Patent Classification (IPC) or to both national classification and IPC

B. FIELDS SEARCHED

Minimum documentation searched (classification system followed by classification symbols)
A61B5/0408, A41D13/12

Documentation searched other than minimum documentation to the extent that such documents are included in the fields searched
Jitsuyo Shinan Koho          1922–1996    Jitsuyo Shinan Toroku Koho    1996–2015
Kokai Jitsuyo Shinan Koho    1971–2015    Toroku Jitsuyo Shinan Koho    1994–2015

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X<br>Y<br><br>A | JP 2-142534 A  (Yokogawa Medical System Ltd.),<br>31 May 1990 (31.05.1990),<br>claims 1 to 2, 5 to 9; page 3, upper left<br>column, line 19 to page 4, upper right column,<br>line 7<br>(Family: none) | 1,15,28-31<br>1-13,15-18,<br>20-21,24-30<br>14,19,22-23 |
| X<br><br>Y<br><br>A | JP 2002-159458 A  (Fukuda Denshi Co., Ltd.),<br>04 June 2002 (04.06.2002),<br>paragraphs [0014] to [0078]; all drawings<br>(Family: none) | 1,15-17,<br>28-31<br>1-13,15-18,<br>20-21,24-30<br>14,19,22-23 |

☒  Further documents are listed in the continuation of Box C.          ☐  See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 13 April 2015 (13.04.15) | 21 April 2015 (21.04.15) |

| Name and mailing address of the ISA/<br>Japan Patent Office<br>3-4-3,Kasumigaseki,Chiyoda-ku,<br>Tokyo 100-8915,Japan | Authorized officer |
| --- | --- |
| | Telephone No. |

Form PCT/ISA/210 (second sheet) (July 2009)

**INTERNATIONAL SEARCH REPORT**

| International application No. |
|---|
| PCT/JP2015/052229 |

C (Continuation). DOCUMENTS CONSIDERED TO BE RELEVANT

| Category* | Citation of document, with indication, where appropriate, of the relevant passages | Relevant to claim No. |
|---|---|---|
| X<br>Y<br><br>A | WO 2005/089642 A1 (Dainippon Sumitomo Pharma Co., Ltd.),<br>29 September 2005 (29.09.2005),<br>paragraphs [0009] to [0018], [0041] to [0042], [0056] to [0057], [0059], [0062], [0066] to [0096], [0101] to [0104]; fig. 1 to 7<br>& US 2009/0012408 A1 & EP 1731090 A1<br>& CN 1933776 A | 31<br>1-13,15-18,<br>20-21,24-30<br>14,19,22-23 |
| Y<br>A | WO 2013/073673 A1 (Nippon Telegraph and Telephone Corp.),<br>23 May 2013 (23.05.2013),<br>paragraphs [0001], [0034]<br>& US 2014/0303470 A1 & EP 2767632 A1<br>& CN 103930612 A | 6-8<br>14,19,22-23 |
| Y<br><br>A | JP 2011-15818 A (Osaka University),<br>27 January 2011 (27.01.2011),<br>paragraphs [0007] to [0011], [0022] to [0042]; fig. 1 to 6<br>(Family: none) | 2-5,9-12,<br>24-27<br>14,19,22-23 |

Form PCT/ISA/210 (continuation of second sheet) (July 2009)

**EP 3 100 677 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013185283 A

**Non-patent literature cited in the description**

- **DAVID M. D. RIBEIRO et al.** A Real time, Wearable ECG and Continuos Blood Pressure Monitoring System for First Responders. *33rd Annual International Conference of the IEEE EMBS,* 2011, 6894-6898